# EUROPEAN PATENT SPECIFICATION

(11) **EP 2 303 225 B1**
(45) Date of publication and mention of the grant of the patent: **20.11.2013**
(21) Application number: 09762157.7
(22) Date of filing: 12.06.2009
(51) Int. Cl.: C07D 477/20, A61K 9/19, A61K 31/407, A61K 9/00

(54) **Compositions of Carbon Dioxide Adduct of Ertapenem and Polymorphic Forms of Ertapenem Monosodium Salt**
Zusammensetzungen des Kohlenstoffdioxid Adduktes von Ertapenem und Polymorphe Formen des Mononatriumsalzes von Ertapenem
Compositions D'Adduit de Dioxide de Carbone D'Ertapénem et Formes Polymorphiques du Sel Monosodium d'Ertapénem

(30) Priority: 11.06.2008 IN DE13852008
(43) Date of publication of application: 06.04.2011
(60) Divisional application: 12172595.6
(73) Proprietor: Ranbaxy Laboratories Limited, New Delhi 110019 (IN)
(72) Inventor: GEORGE, Vinod, Kerala 679333 (IN); VASHISHTA, Bhupendra, Chandigarh Chandigarh 160047 (IN); PRASAD, Mohan, Gurgaon Haryana 122002 (IN); KUMAR, Naresh, Haryana 122018 (IN); ARORA, Vinod, Kumar, Haryana 122101 (IN)
(74) Representative: Cronin, Brian Harold John
(86) International application number: PCT/IB2009/052505
(87) International publication number: WO 2009/150630

(56) References cited:
- WO-A1-01/32172
- WO-A1-97/45430
- WO-A1-98/57973
- WO-A1-99/45010
- WO-A2-03/026572
- WO-A2-03/027067
- WO-A2-2008/062279
- US-A- 5 952 323
- US-A1- 2004 176 351
- US-B1- 6 548 492

## Description

### Field of the Invention

The present invention relatesto process for preparing a carbapenem antibiotic composition.

### Background of the Invention

Carbapenem antibiotics are widely used in treating infections caused by broad range of pathogens including multi-drug-resistant bacteria. Ertapenem sodium is a carbapenem antibiotic, which is chemically a monosodium or a di-sodium salt of 4R-[3(3S*,5S*),4α,5β,6β(R*)]]-3-[[5-[[(3- carboxyphenyl)amino]carbonyl]-3-pyrrolidinyl ]thio]-6-(1-hydroxyethyl)-4-methyl-7-oxo-1- azabicyclo[3.2.0]hept-2-ene-2-carboxylic acid ofFormula I.

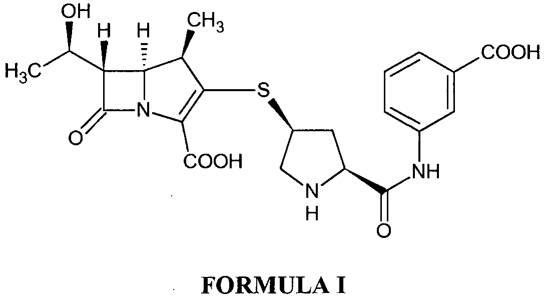

A process for the preparation of ertapenem sodium is provided in U.S.Patent No. 5,652,233 and J. Org. Chem.(2005)70:7479-7487.

U.S.Patent No.6,548,492 says that ertapenem sodium is sensitive to temperature and pH fluctuations.The PCT Publication WO 99/45010 provides a process to purify ertapenem monosodium. According to U.S. Patent No. 6,548,492, ertapenem sodium remains unstable at temperatures above about -20°C. U.S. Patent No. 6,548,492 further says that ertapenem sodium undergoes dimerization and hydrolysis to form undesirable dimers and ring-opened compounds above about -20°C.

As mentioned in U.S.Patent No. 5,952,323, when ertapenem or its sodium salts are formulated in a pharmaceutical composition with a suitable amount of sodium carbonate

or sodium bicarbonate, the compound of Formula II or its salts are formed upon dilution or reconstitution.

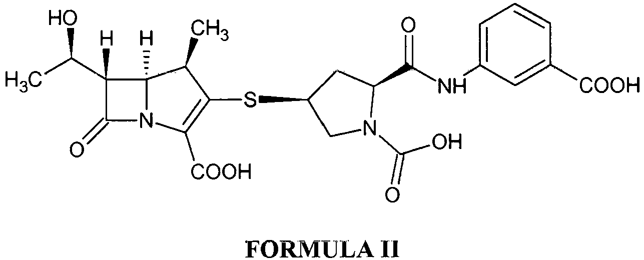

The compound of Formula II or its salts are more stable than ertapenem sodium and the formation of degradation impurities is minimized. Therefore, ertapenem sodium is commercially available as a pharmaceutical composition comprising the compound of Formula II or its salts for parenteral administration.

### Summary of the Invention

The present inventors have developed an advantageous process for the preparation of a pharmaceutical composition comprising the compound of Formula II or its salts. The present inventors have also developed an efficient process for the purification of ertapenem sodium, wherein the process avoids vigorous drying methods using nitrogen sweep or agitation and thereby minimizes the formation of degradation impurities. Thus, the present invention provides an advantageous, efficient and industrially preferable process for the preparation of a pharmaceutical composition comprising the compound of Formula II or its salts.

### Brief Description of the Drawings

Figure 1 depicts the X-Ray Powder Diffractogram (XRPD) of the pharmaceutical composition obtained according to Example 2.
Figure 2 depicts the X-Ray Powder Diffractogram (XRPD) of polymorphic Form D of ertapenem sodium.

### Detailed Description of the Invention

An aspect of the present invention provides a process for the purification of ertapenem sodium, wherein the process comprises,
a) dissolving crude ertapenem sodium in water in the presence of a base,
b) adjusting the pH of the solution obtained in step a) to about 5 to about 6,
c) treating the solution obtained in step b) with one or more alkanols, and
d) isolating the pure ertapenem sodium from the mixture thereof.

Crude ertapenem sodium used as a starting material may contain process related and/or degradation impurities in a quantity not preferable for pharmaceutical use, for example in a quantity of about 4% or more. Crude ertapenem sodium may be obtained during the process of preparing ertapenem sodium according to the prior art, for example, U.S. Patent Nos. 5,652,233 and 7,022,841, and J. Org. Chem.(2005)70:7479-7487, or during the storage of ertapenem sodium obtained by prior art processes. Crude ertapenem sodium is dissolved in water in the presence of a base at a temperature of about 0° to about 10°C. The base may be an alkali metal carbonate or alkali metal bicarbonate, for example, sodium bicarbonate or sodium carbonate. The solution may be optionally further diluted with an alkanol, for example, methanol. The pH of the solution is adjusted to about 5 to about 6, for example, to about 5.5 to about 5.7 at a temperature of about 0° to about 10°C. The pH adjustment may be carried out by treating with an organic or inorganic acid, for example, acetic acid. The solution so obtained may be treated with activated carbon and filtered. The solution is treated with one or more alkanols. The alkanol may be a C₃₋₆ alcohol, for example, n-propanol, or a mixture of a C₃₋₆ alcohol with methanol. The mixture obtained is stirred at about -20° to about 0°C for about 10 minutes to about 100 h. The solid is isolated from the mixture, for example by filtration, and optionally washed with a ketone, for example, acetone, to obtain pure ertapenem sodium. The ertapenem sodium may be further converted into a pharmaceutical composition comprising the compound of Formula II or its salts by the methods known in the prior art or according to the methods disclosed in the present application.

The pharmaceutical composition of the present invention further comprises one or more pharmaceutical excipients including diluents, buffers, preservatives, local anesthetics and tonicity controlling agents. The diluents include sterile water for injection, normal saline, dextrose solution, Ringer's solution and the like. The buffers include dihydrogen sodium phosphate, citrate buffer, meglumine, tri(hydroxymethyl)aminomethane, and the like. The preservative include butylhydroxyacetone, butylhydroxytoluene, benzalkonium chloride and the like. Local anesthetics include benzocaine, lidocaine, novacaine, pontocaine and the like. Tonicity modifying agents include sodium chloride, mannitol, dextrose, glucose, lactose, sucrose and the like.

The purity was determined by high-performance liquid chromatography(HPLC) method using ACE C 8, 5µ column and UV detector (250 nm). Gradient system Acetonitrile: Buffer (Ammonium acetate/ Acetic acid pH 6.5) was used as a mobile phase and the flow rate was 1.0 ml/minute.

XRPD of the samples were determined by using Panalytical X'Pert Pro X=Ray Powder Diffractometer in the range 3 to 40 degree 2 theta with a step size of 0.02 and under tube voltage and current of 45 Kv and 40 mA respectively. Copper radiation of wavelength 1.54 angstrom and Xceletor detector were used.

While the present invention has been described in terms of its specific embodiments, certain modifications and equivalents will be apparent to those skilled in the art.

### EXAMPLE

### Preparation of Polymorphic Form D of Ertapenem Monosodium

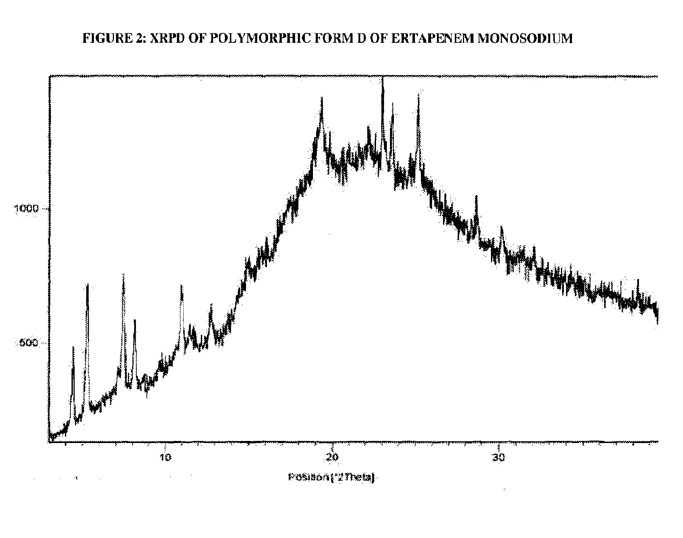

Ertapenem sodium (50 g, HPLC Purity: 95%) was dissolved in aqueous sodium bicarbonate solution (9.3 g in 400 mL) at 0°C to 5°C. Methanol (100 mL) was added to the solution and the pH of the solution was adjusted with a mixture of acetic acid and methanol (1:1) to 5.5 to 5.7 at 0°C to 5°C. The solution was diluted further with methanol (100 mL) and treated with activated carbon (5 g). The mixture was filtered and washed with a pre-cooled (0°C to 5°C) mixture of water (100 mL) and methanol (50 mL). The combined filtrate was cooled to -5°C to 0°C and n-propanol (250 mL) was added to the cooled solution, followed by the addition of seed ertapenem monosodium (0.5 g; obtained by following the present example without employing seed) at about -5°C. The mixture was stirred at -10°C to -5°C for 15 to 20 minutes. A mixture of n-propanol (750 mL) and methanol (750 mL) was added slowly into the mixture in 90 to 100 minutes at -15°C to -10°C and stirred for 2 hours to 3 hours at - 15°C to -10°C. The mixture was filtered, washed with acetone (100 mL) and dried under vacuum for 30 minutes in nitrogen atmosphere to obtain the title compound (MW: 497.5) as a free flowing solid having an XRPD pattern as depicted in Figure 2 of the accompanied drawing.
Yield: 30 g
HPLC Purity: 98 %
Sodium Content: 4.3% w/w (by ISE method, on anhydrous basis)

### Example 2: Preparation of Sterile Carbapenem Antibiotic Composition

Polymorphic Form D of ertapenem monosodium (27.5 g) as prepared in Example 1 was added slowly to a solution of sodium bicarbonate (4.0 g) in water (100 ml) at 0° to 5°C with simultaneous addition of aqueous sodium hydroxide solution (10 % w/v) to maintain the pH of the solution between 7.4 and 7.7. The solution so obtained was filtered through a 0.2 micron membrane filter, loaded on a lyophilization tray in a tray lyophilizer and lyophilized according to the conditions provided below:

| **STEP** | **STEP TYPE** | **TEMPERATURE (°C)** | **VACUUM (mTorr)** | **TIME (h)** |
|---|---|---|---|---|
| 1 | Freezing | -40 | - | 2 |
| 2 | Freezing | -40 | - | 2 |
| 3 | Evacuation | - | 100 | - |
| 4 | Drying | -10 | 100 | 3 |
| 5 | Drying | 0 | 100 | 2 |
| 6 | Drying | 0 | 100 | 15 |
| 7 | Drying | 10 | 100 | 1 |
| 8 | Drying | 10 | 100 | 8 |
| 9 | Drying | 15 | 100 | 1 |
| 10 | Drying | 15 | 100 | 7 |
| 11 | Drying | 20 | 100 | 1 |
| 12 | Drying | 20 | 100 | 7 |
| 13 | Drying | 25 | 100 | 1 |
| 14 | Drying | 25 | 100 | 5 |
| Total Time | | | | 55 |

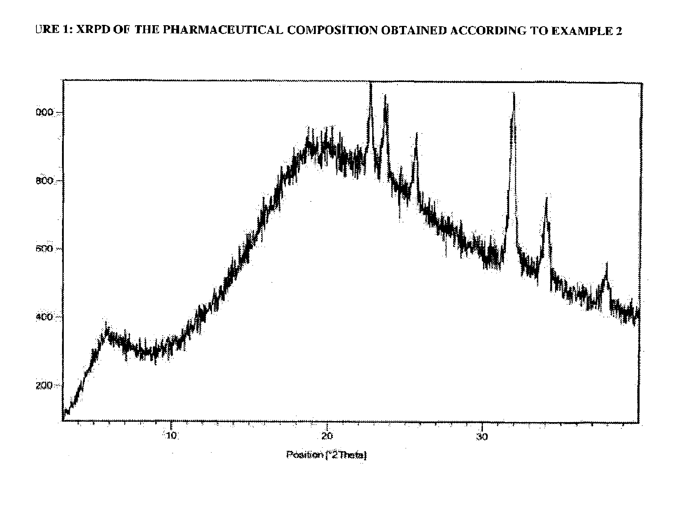

The sterile lyophilized pharmaceutical composition of the sodium salt of the compound of Formula II (Molecular Weight: 585.5) having an XRPD pattern as depicted in Figure 1 of the accompanied drawing was unloaded from the lyophilization tray.
Yield: 27.7 g
HPLC Purity: 97%
Dimer I and Dimer II: 0.70%
Dimer III: 0.2%
Dimer-H₂Oa and Dimer-H₂Ob: 0.45%
Dimer V: Not detectable
Ring Opened Impurity: 1.0%
Sodium Content: 11.0% w/w (by ISE method, on anhydrous basis)

## Claims

1. A process for the purification of ertapenem sodium, wherein the process comprises,
a) dissolving crude ertapenem sodium in water in the presence of a base,
b) adjusting the pH of the solution obtained in step a) to 5 to 6,
c) treating the solution obtained in step b) with one or more alkanols, and
d) isolating the pure ertapenem sodium from the mixture thereof.

2. The process of claim 1 wherein the pure ertapenem sodium is further converted into a pharmaceutical composition comprising the compound of Formula II or its salts,

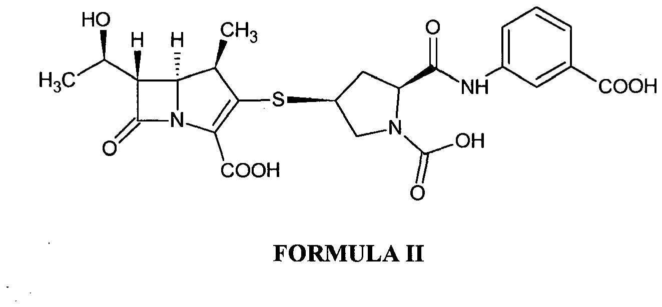

## Patentansprüche

1. Verfahren zur Reinigung von Ertapenem-Natrium, wobei das Verfahren
a) das Lösen von rohem Ertapenem-Natrium in Gegenwart einer Base in Wasser,
b) das Einstellen des pH der in Schritt a) erhaltenen Lösung auf 5 bis 6,
c) das Behandeln der in Schritt b) erhaltenen Lösung mit einem oder mehreren Alkanolen und
d) das Isolieren von reinem Ertapenem-Natrium aus dessen Gemisch umfasst.

2. Verfahren nach Anspruch 1, wobei das reine Ertapenem-Natrium zu einer pharmazeutischen Zusammensetzung weiter verarbeitet wird, welche die Verbindung der Formel II oder deren Salze umfasst

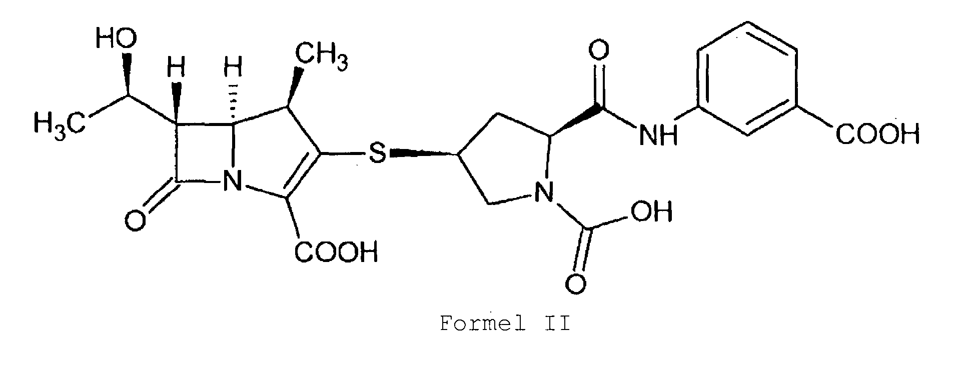

## Revendications

1. Procédé pour la purification de sodium ertapénème, procédé qui comprend les étapes qui consistent à :
a) dissoudre le sodium ertapénème à l'état brut dans de l'eau en présence d'une base ;
b) ajuster le pH de la solution obtenue à l'étape a) entre 5 et 6 ;
c) traiter la solution obtenue à l'étape b) avec un ou plusieurs alkanols ; et
d) isoler le sodium ertapénème pur à partir dudit mélange.

2. Procédé selon la revendication 1, dans lequel le sodium ertapénème pur est, en outre, converti en une composition pharmaceutique contenant le composé de Formule II ou ses sels.
